(19) **Europäisches Patentamt / European Patent Office / Office européen des brevets**

(11) **EP 4 176 922 A1**

(12) **EUROPEAN PATENT APPLICATION**

(43) Date of publication:
**10.05.2023 Bulletin 2023/19**

(21) Application number: **21383010.2**

(22) Date of filing: **08.11.2021**

(51) International Patent Classification (IPC):
**A61M 60/178** (2021.01)   **A61M 60/50** (2021.01)
**G16H 20/40** (2018.01)   **G16H 40/60** (2018.01)
**G16H 50/50** (2018.01)

(52) Cooperative Patent Classification (CPC):
**A61M 60/50; A61M 60/178; A61M 60/183;
A61M 60/216; G16H 20/40; G16H 40/60;
G16H 50/50**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ELEM Biotech S.L.
08029 Barcelona (ES)**

(72) Inventors:
• **SANTIAGO, Alfonso
Minneapolis, MN 55413 (US)**
• **VÁSQUEZ, Mariano
08029 Barcelona (ES)**

(74) Representative: **Keltie LLP
No. 1 London Bridge
London SE1 9BA (GB)**

(54) **PERFORMANCE OPTIMISATION OF VENTRICLE ASSIST DEVICES**

(57)    A method of determining effects of starting or operating a ventricular assistance therapy, such as a ventricular assist device, is described. One or more parameters of a patient heart and one or more parameters of the ventricular assistance therapy are determined. A mechanical and fluid dynamic model of at least a ventricle where the ventricular assistance therapy is or is to be used is then used to compute predicted quantities of interest for the patient heart from starting or operating the ventricular assistance therapy. This may be carried out iteratively until a predetermined configuration objective is achieved.

Figure 1

EP 4 176 922 A1

**Description**

FIELD OF DISCLOSURE

Ventricular assist devices (VADs) are implantable artificial pumps which aim to partially or fully replace function of the heart. VADs are most frequently designed to assist the left ventricle (LVAD), but they can be used to assist the right ventricle (RVAD) or both ventricles (BiVAD).

BACKGROUND TO DISCLOSURE

VADs provide lifesaving therapy for patients with advanced heart failure and are used as a bridge to a future heart transplant or as a destination therapy (where the reliance on VAD is intended to be permanent). Despite improved survival, multiple side-effects are associated with these therapies. Major complications still occur, as bleeding or thrombosis, occurring in about 20% of cases [4, 6].

One significant issues is that changes in flow pattern in the left ventricle (LV) resulting from VAD use may induce stagnant regions in the LV. Later generations of LVADs have introduced "artificial pulse" patterns, a modulation of pump speed that tries to disrupt the stagnant regions. This is not however a full solution to the problem, and a number of other variables in the implantation of VADs have been shown to affect thrombosis risk, in particular, cannula alignment and placement [10, 11]. However, it is difficult to determine the success or failure of such approaches, or to identify the best conditions of VAD installation or operation for an individual, without a trial-and-error approach.

There is also evidence [2] of inflammation associated with VAD use, which leads to an increased risk of thrombosis. This, combined with risks of endothelial lesion and the described abnormal flow patterns [5] are the three component parts of the Virchow's triad [3] for thrombus formation. The local flow conditions also influence the type of thrombus created. High velocities and high shear stresses lead to platelet activation [8] which aggregates fibrin, therefore forming so-called white thrombi. In contrast, stagnant and slow recirculating flows and low shear stresses lead [9] to an aggregation of all blood components including erythrocytes as well as leukocytes infiltrated with fibrin [7] leading to so-called red thrombi.

In this context, VAD are generally composed by a centrifugal or axial pump that removes blood from the ventricle by suction. The highest speeds are produced in the surroundings of the rotor. The lowest speeds are produced in the far field of the suction domain (in this case the LV itself). Therefore, white thrombi are created in the rotor vicinities while red thrombi are created in the far field of the suction domain (LV). The problem of white thrombus formation is generally addressed by appropriate pump design - for example, by use of hydrodynamic or magnetic bearings.

After the flow has been diverted from the ventricle to the inside of the cannula pump, the rotor and the remainder of the flow path to the exiting graft to connect with the aorta, it becomes a purely engineering problem which is thought to be affected by a patient's anatomy or condition only to a negligible effect. The main practical difficulty that is still to be addressed effectively is that of red thrombus formation in the LV itself.

In particular, it is difficult to obtain reliable data on the efficacy of VAD approaches. While clinical trials and animal studies of VADs are an accurate representation of reality, they involve:

- high costs.
- low (often non-statistically relevant) number of samples.
- Little or no control over patient parameters.
- Ethical issues.
- Little or no access to critical measurable information (e.g. fluid dynamics).

Bench tests of VADs are found to have some advantages and disadvantages compared to clinical studies (or numerical modelling), but involve:

- Requirements for expensive low-use equipment.

- Poor control over variables.

- Scarce measurements.

- User error.

In contrast, computer simulations are cheaper than clinical, animal and bench studies, do not require any specific equipment, allow a high level of control on variables, and permit obtaining quantities of interest in high levels of detail.

SUMMARY OF DISCLOSURE

In a first aspect, the disclosure provides a method of determining effects of starting or operating a ventricular assistance therapy, comprising: determining one or more parameters of a patient heart; determining one or more parameters of the ventricular assistance therapy; and using a mechanical and fluid dynamic model of at least a ventricle where the ventricular assistance therapy is or is to be used, computing predicted quantities of interest for the patient heart from starting or operating the ventricular assistance therapy.

This method may involve varying one or more of the parameters, and then continuing the computation step for varied parameters until a set of determined parameters and associated predicted quantities of interest have been obtained to meet a predetermined objective. This objective may be to identify an operating space across a range of variable values. The objective may be to determine parameters of ventricular assistance therapy leading to predicted quantities of interest meeting predetermined criteria (such as indices for performance, safety and effectiveness). The ventricular assistance therapy may be implantation and operation of a ventricular assist device, for example a left ventricle assist device.

While in principle parameters from a patient heart may be obtained for a specific patient, an approach of particular interest is to determine these parameters for an uncertain population. Parameters of the patient heart may relate to a subject's anatomical geometry or cardiac activity (for example, heart morphology and other subject specific geometry, ventricular volumes such as end diastole volume, ejection fraction and heart rate) or to a subject's systemic condition (such as arterial mean pressure, arterial resistance or arterial capacitance). Parameters of the ventricular assistance therapy, for a VAD, may include a subject cannula implantation specification (position of implantation, depth of insertion, cannula angle, cannula shape, cannula geometry) or the VAD pump operation condition (pump speed waveform, pump synchronisation with native heart rate, pump performance function (H-Q function)).

Quantities of interest may result directly from the computation (for example, ventricular velocity and pressure fields) or may be derived (such as residence time, velocity magnitude, kinetic energy, distortion time and pulsatility index). A predetermined condition may relate for example to avoiding regions with high risk of thrombus formation - these are characterised by one or more of high residence time, low kinetic energy, high distortion time and low pulsatility index.

Computation may take place over two meshes - a solid mechanics mesh determined from an original heart geometry and a fluid dynamics mesh created by extruding inlets and outlets for flow development from the solid. Computation may involve use of the Navier-Stokes equations.

Accordingly, provided are a framework of tools aiming to enable accurate computer models of VAD therapeutic approaches, in particular allowing for:

- The creation of populations of patients with different stages of heart failure
- The application of user-defined pump modulation protocols
- The measurement of Quantities of Interest (QoIs) related to fluid stagnation
- The measurement of QoIs related to pressures and stresses on the valves and ventricle walls
- Iteration over the model in order to achieve one or more of the following:

  - optimise speed modulation protocol for an uncertain population
  - find optimal speed modulation for a patient
  - find optimal cannula shape angulation and position for a patient

These tools can be used to enable the VAD to be implanted and configured most effectively - for example for an LVAD, by appropriate cannulation of the left ventricle in providing the fluid channel to the LVAD pump - and by pre-configuring VAD operation (particularly speed modulation) for suitability for the patient.

CONTEXT OF DISCLOSURE

It should be noted that while LVADs are considered in detail here, the approaches described above may be applied to VADs more generally (and so for RVADs and BiVADs, for example). Consequently the term VAD is used generally below, though this will in practice be an LVAD where any issues that relate specifically to an LVAD are discussed.

It is helpful for context to consider a standard process for configuration of a VAD. After installation of an VAD, optimization of VAD speed is routinely performed for a patient post-implant using a ramp study. Transthoracic echocardiography measurements of cardiac geometry and function are made while VAD speed is slowly increased over a wide range. The final pump speed is selected by balancing overall cardiac output, efficiency of left ventricle (LV) unloading, and preserving flow pulsatility. Several variables are assessed from standard echo views including LV end-diastolic dimension, LV end-systolic diameter, frequency of Aortic Valve (AoV) opening, degree of valve regurgitation, right ventricle (RV) systolic pressure, blood pressure, and heart rate (HR) at each speed setting. In addition, VAD pump power, pulsatility index and flow are recorded. For example, for a Thoratec HeartMate II, the ramp speed protocol starts at a speed of 8k[rpm] and increases by 400[rpm] increments every 2 minutes until a speed of 12k[rpm] is reached. As LVAD speed is increased, the LV volume decreases, as does the frequency of AoV opening and pulsatility of flow. Excessive LV unloading at higher LVAD speeds increases the demand on the right heart, causing tricuspid regurgitation and can also produce suction events which disrupt the flow into the LVAD inflow cannula.

The clinical practice for LVAD speed selection first ensures that the hemodynamics are compatible with life, e.g. a mean arterial pressure greater than 65 mmHg and a minimum cardiac index of 2.2[L/min/m2] of body surface area (BSA). To optimize LV unloading, the interventricular septum position should not bow towards either the left or right. If these conditions are met, the LVAD speed is selected that achieves intermittent AoV opening while maintaining no more than mild mitral regurgitation or aortic insufficiency. *De novo* AoV insufficiency development in LVAD patients is linked to lack of AoV opening. Interestingly, AoV insufficiency occurred in the majority of LVAD patients (66%) whose AoVs remained closed during support, but rarely (8%) in those whose AoVs opened regularly.

A patient-specific LVAD speed calibration is important for ensuring appropriate cardiovascular support and for minimizing the frequency of adverse events related to long-term support. However, the ramp echo study is not performed routinely after the first month post-implant, due to the expense and inconvenience. A computational tool that predicts cardiac output and aortic valve opening for the subject's characteristics can therefore reduce the ramp study requirements as well as contribute to speed adjustments required over a long-term, even supporting a speed adjustment paradigm that contributes to recovery. A computational tool can be used with the highest risk devices to ensure that the LVAD speed selection is in an effective range from installation, minimizing the likelihood that a significant correction is needed as a result of the ramp study and so reducing risk to the patient.

## DETAILED DESCRIPTION OF EMBODIMENTS

Specific embodiments of the invention will now be described, by way of example, with reference to the accompanying figures, of which:

Figure 1 provides an overview of a model for use in embodiments of the invention illustrating the role of parameters and quantities of interest;

Figure 2 shows an exemplary computing architecture for implementing embodiments of the invention;

Figure 3 is a scatter plot showing a comparison between numerical results according to implementations of the invention and experimental results; and

Figures 4A to 4C show box and scatter plots for a population of 9 patients for three different pump speed modulation protocols, with Figure 4A showing results for aortic flow, Figure 4B showing results for kinetic energy, and Figure 4C showing results for pulsatility index.

We will now describe below the physical basis and computational implementation of a computational tool for modelling left ventricular flow after LVAD implementation which can be used for effective LVAD installation and initial configuration - while relevant to VADs more generally, specific application to LVADs is shown here. We also show below in outline steps needed to demonstrate that the computational model is fit for purpose. The V&V 40[1] standard provides a framework for assessing the relevance and adequacy of the completed verification, validation and uncertainty quantification (VVUQ) activities that establish the credibility of a computational model. The standard requires as a first step to identify (1) the question of interest, this is the question the tool will find an answer to; (2) the context of use (CoU), this is the specific role and scope of the computational model; (3) the quantity of interest (QoI) these are the measurements relevant for the CoU that will be used for the prediction; (4) the model influence, this is the contribution of the model in making a decision; and (5) the model risk and decision consequence, this is the possibility that incorrect model results might lead to patient harm.

### Context of Use (CoU)

The heart-LVAD computational model may be used to assist in the preclinical design and development of LVAD, by identifying operational conditions that might lead to stagnant ventricular flow. This can therefore be used to ensure that baseline operating criteria are chosen to be safe for the general population - it can also be used to provide advance probabilistic tuning based on patient specific data if this has been used in the model. This is shown here by code and numerical verification by computing the observed rate of convergence in a manufactured solution and a mesh convergence study; uncertainty quantification (UQ) with mixed aleatory-epistemic inputs using validation against a bench experiment with twelve operation conditions.

The heart-LVAD computational model will then be used to extract quantities of interest (Qols) related to flow stagnation and that highlight regions that may be related to thrombus formation (relevant properties include, but are not limited to, stagnation time, kinetic energy and distortion). Unlike animal experiments, the proposed model provides insight on severe heart failure working conditions.

Input parameters

It is contemplated that approaches as described herein may include further input parameters which are personalised to a particular subject and/or which reflect a proposed therapeutic approach. Parameters of relevance include those that characterise the patient geometry, the pump implantation and the operation of the pump. Such inputs can improve the accuracy of the produced models and therefore improve the measurements which can be derived from the models, and the confidence of any recommended therapeutic actions.

Example input parameters which may be used include the below which may be used independently or in any combination. These parameters can be identified from specific subjects, can be determined by the user, and/or can be generated from population data. For example, known average values for a population can be used, where a population can be a general healthy population, or a population selected for various criteria such as disease state, age, sex, and so on.

A. Subject's anatomical geometry and/or parameters related to cardiac activity

A.1. subject specific geometry (for example, heart morphology, and trabeculations)
A.2. ventricular volumes, such as end diastolic volume (EDV)
A.3. ejection fraction (EF)
A.4. heart rate (HR)

B. Subject systemic condition:

B.1. Arterial mean pressure
B.2. Arterial resistance
B.3. Arterial capacitance

C. Subject cannula implantation specification

C.1. cannula position implantation
C.2. cannula insertion depth
C.3. cannula angle
C.4. cannula shape
C.5. Cannula geometry (for example, cannula diameter)

D. Pump operation condition

D.1. time-variant pump speed waveform (including constant speeds)
D.2. time-variant pump speed synchronisation point with the native heart rate.
D.3. Pump performance function characterisation by speed (also referred to as H-Q function, where H is pressure head and Q is flow).

Use of a number of these variables is discussed further below in the context of specific implementations. It should be noted that the list above is non-limiting.

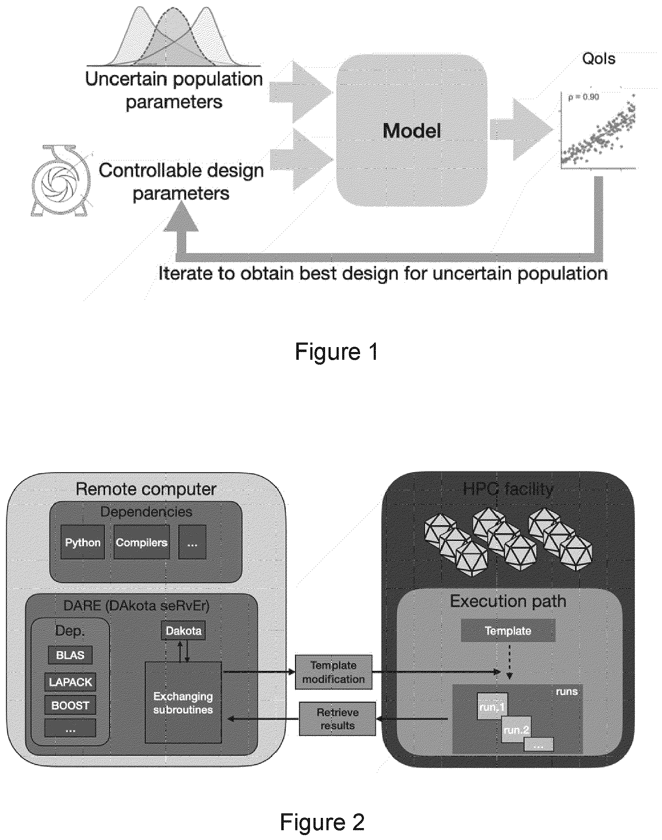

Here, it should be noted that the parameters in sections A and B are properties of a subject, and they will thus not themselves form part of an output from a modelling process. The parameters in sections C and D, by contrast, are features that can be varied or controlled in implementation as a part of the implantation process (section C) or by selection or control of the pump (section D). The relationship between parameters, the model, and the quantities of interest is shown in Figure 1.

Outputs: Quantities of Interest (Qol)

Models as described herein produce Qol which can be directly measured from the models or can be provided by further processing these measurements. The Qols are:

A. Raw Qols: In the model shown below, these are the Qols extracted immediately after the solution of the Navier-Stokes equation. Modifications to the model including other structural elements or physical considerations (such as electrophysiology) will involve solution to other equations and may lead to additional or even different Qols. These require added post-processing steps (items B, C, D...) to extract relevant information for industrial and clinical use.

    A.1. Ventricular velocity field
    A.2. Ventricular pressure field

B. Integrated or Derivative Qols (for example, volumetric Qols): Operations executed with the velocity and pressure fields that are volumetrically calculated and that augment the information obtained.

    B.1. Residence time: Time spent by a fluid particle in the domain.
    B.2. Magnitude of velocity: point-wise root mean square of the flow velocity.
    B.3. Kinetic energy: point-wise root mean square of the flow velocity, multiplied by the fluid density.
    B.4. Distortion time: Measure of the flow strain obtained from the first invariant of the symmetric strain tensor.
    B.5. Pulsatility index (maximum flow velocity variations in a localised area, where high pulsatility indices may signify greater acceleration/deceleration of blood)

C. Curve Qols: Volumetric integrals of the Integrated or Derivative Qols (B) that allows reducion of the 3D results to time-variant curves. They represent the behaviour in a region of interest (ROI). For instance, ROI for these Curve Qols could be the whole LV, the cannula pocket and the aortic root region, but also any other region that might be a potential zone of thrombus formation, including a single localization.

    C.1. averaging Qols: average of the QoI in the ROI for each time step.
    C.2. maximum/minimum Qols: maximum and minimum in the domain for each time step.

D. Scalar Qols: single-number representations of the curve QoI. Obtained by some kind of time integration of the curve Qols during a specific time window.

    D.1. Time-averaging scalar Qols: average of the curve QoI between some time bounds (for example, 1.23[s] to 3.24[s] - the time window may be systole, or may be up-phase of the LVAD speed modulation)
    D.2. max/min scalar Qols: minimum and maximum values of the curves.

Curve and scalar Qols can be used as a way of reducing the complexity of the results, increasing the ability of detecting differences compared to a video output. Each volume QoI has an associated Curve QoI. For example, Kinetic energy outputs have a field, curve and scalar representation.

<u>Correspondence with physical outcomes and selection of optimal approach</u>

From the above QoI, it is possible to identify characteristics in an output model which can provide information about the likely outcome of a given VAD approach, such as regions of stagnation. Stagnant regions, and therefore regions with high risk of thrombus formation are regions with one or more of:

    A) High residence time
    B) Low kinetic energy
    C) High distortion time
    D) Low pulsatility index

If comparing two devices or proposed approaches, the "best" device or approach will be the one that demonstrates the opposite, namely:

    A) Lower residence time
    B) Higher kinetic energy
    C) Low distortion time
    D) High pulsatility

Numerical model credibility

While VVUQ is not mandatory to use the simulation result for design guidance or as regulatory evidence, it certainly increases the trustworthiness of the results. For example, ASME V&V40 [1] requires experimental data to compare simulation results and ensure credibility.

An easily accessible and reproducible bench experiment is a particularly effective way to assess model credibility. Such a bench experiment could involve at least an idealised ventricle model made of a flexible material including a connection tubing for the VAD. Such a bench experiment should be able to obtain flow and pressure measurements in the inlets, outlets, and cavity as well as particle image velocimetry (PIV) of the cavity. These devices are known as pulse duplicators, and the reader might find a commercial option in the following link: https://vivitrolabs.com/product/pulse-duplicator/.

Working principles of model

An exemplary analysis is presented below.

A Sequence for a single analysis:

1. Create the baseline patient or baseline population by selecting the ventricle end-diastolic volume (EDV), ejection fraction (EF), and heart rate (HR). If the prediction is for a single patient, these parameters are chosen as single values. If the prediction is for a population, the parameters are characterised as probability distributions and afterwards the distributions sampled to obtain the sample of patients from the population to calculate.

2. The heartbeat for the patient(s) is computed through a fluid-structure interaction (FSI) simulation that deforms the ventricular mesh to fit the patient-specific parameters. This approach can be made accurate yet computationally relatively inexpensive. This technique can also be used to modify other geometrical features as cannula insertion or angulation, or any other input parameters as previously described. In order to modify the cannula position and/or shape, a new geometry can be used, or the original geometry could be deformed by using displacement boundary to obtain the desired geometrical configuration.
The FSI in this embodiment involves construction of two meshes. The solid mechanics mesh is created directly from the original geometry. The CFD mesh is created by closing the solid domain and extruding the inlets and outlets to ensure flow development. The mesh is spatially discretised using linear tethrahedra, pyramids and pentagons. For the time discretisation, a first order trapezoidal rule is used.

3. Describe the treatment to apply. This includes:

a. time-variable pump speed (i.e. speed modulation). This is what is the magnitude of the baseline speed and how many, how large, in which direction (upstroke or downstroke), and how frequently are the speed changes.
b. If used in the speed modulation, provide the synchronisation point with the native heart rate (e.g. cycle upstroke happens during isovolumetric contraction).
c. H-Q curves that describe the pump performance in the operation conditions. This includes the HQ curves for, at least the extreme cases in the operation conditions. The intermediate H-Q curves will be automatically obtained through a linear interpolator.

4. Select speed modulation and pump characteristics (i.e. the treatment to apply). This is done through an H-Q performance curve of the pump (H-Q being a graphical expression of how much flow (Q) can be generated due to the increased head/pressure (H) which is added to the fluid by the pump). There is a single H-Q curve for each pump speed and afterwards the H-Q curves are linearly interpolated to find the unknown speeds.

5. Execute the simulation(s). The simulation engine is based on the finite elements method (FEM) and involves the incompressible Newtonian Navier-Stokes equations using an Arbitrary Lagrangian-Eulerian formulation that allows deforming the mesh in the fluid problem.

$$\rho \frac{\partial v_i}{\partial t} + \rho \left( v_j - v_j^d \right) \frac{\partial v_i}{\partial x_j} + \frac{\partial}{\partial x_j} \left[ +p\delta_{ij} - \mu \left( \frac{\partial v_i}{\partial x_j} + \frac{\partial v_j}{\partial x_i} \right) \right] = \rho f_i$$

and

$$\frac{\partial v_i}{\partial x_i} = 0,$$

where $\mu$ is the dynamic viscosity of the fluid, $\rho$ the density, $v_i$ the velocity, p is the mechanical pressure, $f_i$ the volumetric force term and $v_j^d$ is the domain velocity. As the fluid domain deforms due to imposed boundary displacements, the deformation for the inner nodes is computed through a diffusion equation. The solving domain includes the intraventricular fluid and the inflow/outflow conducts.

The Mitral inlet has a constant pressure of $P_{LA}$. The aortic model has a 0D Windkessel model with three components: a resistor $R_s$, serially connected with an RC parallel impedance $R_p$, and $C_p$. On the deforming ventricular walls, as well as on the rest of the fluid domain, the velocity of the domain deformation is imposed. This is $v_i|\Gamma_d = \partial b_i/\partial t$,

where $v_i|\Gamma_d$ is the velocity at the deformed boundary.

A pressure dependent flow boundary condition is provided at the LVAD outflow as will be described further below. The rest of the boundaries have $v_i|\Gamma_r = 0$, where the initial fluid velocity is $v_i|_{t=0} = 0$

Mitral and aortic valves are modelled through a pressure-driven porous layer in the valvular region. This porous media add an isotropic force to the right-hand side of the momentum equation with the shape $f_i^P = \sigma_{ij}^P v_j$, where

$\sigma_{ij}^P = PI_{ij}$, where P is the material porosity and $I_{ij}$ the identity matrix. This strategy provides a robust numerical scheme against the potential ill-conditioned stage of confined fluid. To ensure a smooth change with potentially abrupt changes in the transvalvular pressure, the porosity is driven through a hyperbolic tangent as:

$$P = P_{max}\left[1 + \tanh\left(\frac{\Delta p^V - \Delta p_{ref}^V}{s}\right)\right],$$

where $P_{max}$ is the maximum possible porosity, s is the slope of the curve, $\Delta p^V$ is the transvalvular pressure drop, and $\Delta p_{ref}^V$ is a reference pressure gradient. In practice, if $\Delta p^V \gg \Delta p_{ref}^V$, the valve is closed, and if $\Delta p^V \ll \Delta p_{ref}^V$, the valve is open. To avoid spurious valve opening and/or closing due to transient peaks in the transvalvular pressure gradients, the measures are filtered using a median filter.

For the LVAD boundary condition, a pressure-flow transfer function is used which will be given by the characteristic performance curve of the pump at a determined speed. These pressure-flow curves (also called H-Q curves) provide a relation between the pressure difference between the pump inlet and outlet, and the flow the pump can provide at that speed. If the pump speed changes during the simulation as a consequence of the time-variable speed, the correspondent H-Q curve for that speed is used.

If $\Delta p^{VAD} = P_{Ao} - P_{LV}$ is the pressure difference between the outlet and the inlet of the pump and $Q_{VAD}$ the flow through the pump, then the pressure-flow relationship can be approximated as follows:

$$\Delta p^{VAD} = a_{VAD} + b_{VAD}Q_{VAD} + c_{VAD}Q_{VAD}^2$$

For each $\Delta p^{VAD}$ there is a single $Q_{VAD}$ and vice versa - this relation can be used as a boundary condition, imposing a flow rate for a calculated pressure difference $\Delta p^{VAD}$ - in this way the flow rate is constrained to meet this equation.

As indicated above, the aortic outlet is coupled with a Windkessel (second order R-RC model) that models the lumped systemic artery. Reverse flow at outlets and inlets is stabilised through a high viscosity layer.

6. Extract the quantities of interest (Qols). The Qois are calculated on-the-fly together with the volumetric averages, means, maxima, minima and standard deviations for the multiple region of interest (ROI) and time windows. These were listed above and can be defined as follows:

a. Raw and Derived Qols:

i. Velocity field - The velocity on each point of the domain is the solution of the Navier-Stokes equation, and the quantity that determines the ventricular fluid motion. It is also used to calculate the kinetic energy (K), the residence time $T_R$, the distortion time $T_D$ and the pulsatility index.

ii. Pressure field: the pressure obtained after enforcing continuity in the Navier-Stokes equation.

iii. Residence time field - This is the time spent in the domain of interest by a fluid particle. Regions with high TR are potentially risk areas for red thrombus formation, but it does not necessarily imply risk of thrombus. E.g. vortices are regions with comparable high residence time, but they do not produce a thrombus formation risk. Therefore, the $T_R$ needs to be considered in conjunction with other quantities like the kinetic energy (K) and the distortion time ($T_D$).

iv. Distortion time field - This is a measure derived from the strain tensor with time units, representing the time required by the fluid to shear. A laminar flow has a high $T_D$ as there is little shear, while a highly turbulent flow has a small $T_D$ as vortices produce high shear with viscous energy dissipation.

v. Kinetic energy field - This is calculated as $K = \frac{1}{2}\rho v^2$, where $\rho$ is the fluid density and v is the magnitude of the velocity. This is a measure of the fluid energy. High kinetic energy regions, while might have also high residence time, do not involve thrombus risk. However, K is not a Galiliean invariant, so there might be regions of high K with little to no distortion (e.g. bolus of fluid moving from the atria to the ventricle) therefore this may need to be considered with a measure of vorticity.

vi. Pulsatility index - This is a volumetric, beat-wise measurement. For each beat, on each point of the domain, the maximum $v_{max}$, minimum $v_{min}$, and average $v_{avg}$ velocities are computed using a predetermined sampling frequency. Afterwards for each point of the domain the pulsatility index is computed as

$$PI = (v_{max} - v_{min})/v_{avg}$$

and obtained beat-by-beat. The PI is an index for fluid velocity variation, therefore a stationary flow would have PI = 0[-] while a highly transient flow has a PI » 0[-].

7. These raw and processed Qols are integrated in volume and or time to obtain the correspondent curve and scalar Qols that predict the stagnation regions induced by the particular patient-device combination.

It should be noted that the approach described above is exemplary, and it may be varied in a number of respects. One factor that can be addressed in a number of ways is the movement of ventricular (endocardium) walls. To achieve this, a position vs. time series may be imposed at each node/element of the VAD problem surface mesh of the endocardium, using an appropriate interpolation algorithm. This series may be expressed as a function, or it may be provided as a table. There are number of ways in which this could be derived:

- As a one-way scheme derived from patient images showing wall motion;
- As a one-way scheme derived from electromechanical simulations - ventricular mechanical motion is driven by electrophysiology, and a coupled electromechanical simulation is used to obtain wall motion;
- As a one-way scheme derived from solid mechanics simulations - in this case, ventricular mechanical motion is driven by pericardium pressure forces;
- As a two-way scheme from electromechanical simulations - a separated electromechanical-fluid mechanics problem is solved, and the resulting endocardium wall motion used to impose the motion of the VAD problem surface mesh on the endocardium; and
- As a two-way scheme with a fully coupled electromechanical-fluid mechanics problem solved on the same domain as the VAD problem.

B) Automatising tool. The single simulation defined in (A) is executed in an HPC environment as a cluster and treated as a building block of the larger simulation process from now on - this larger simulation process treats this building block as effectively a black-box. In the exemplary approach shown here (see Figure 2), an external system (DARE, DAkota SeRvEr) is in charge of automatising the black-box execution from one or multiple input parameters and return the potential results. More generally, the automatising tool automatises the job configuration, execution and result retrieving by reading the patient/device combination input samples (even for a single patient or for a population) and creating job input files that are posteriorly submitted to the HPC environment for execution. The process starts with a template set of files that contain flags that are replaced by the automatising tool with the final parameters. Once the inputs are created, files are exchanged with the HPC environment, and the job submitted to the queue. The HPC queue manager is in charge of assigning priorities to each task. After submitting a job, the automatising tool waits a variable (depending on job size and length) amount of time before asking for a successful job termination. Once the job is finalised the automatising tool executes the user defined post-processing script (e.g. the script may contain a set of instructions to extract a single column from an output file) and retrieves the final scalars. These scalars may be the final desired result or be part of an optimisation loop. Multiple instances of the automatising tool can be launched in parallel allowing full parallel efficiency. Robustness is obtained via implementation of SSH (secure shell) exception handling to avoid termination due to a potentially faulty connection.

The actions of the automatising tool are:

- Create the remote (cluster) and local directory tree.
- Create symbolic link to the template files.
- Modify the required template files.
- Launch the execution.
- Wait for completion and/or detect errors
- Execute postprocessing script
- Retrieve results

C) Sampling tool. An exemplary sampling tool is Dakota (Sandia), but any other suitable sampling tool may be used to select the new parameters for the execution that the automatising tool will use to configure and execute the simulation. The sampling tool needs to:

- Select parameters from range/distribution.
- Optimise inputs to obtain desired output if required.
- Gather results
- Create surrogate models for performance with stochastic collocation (SC), polynomial chaos expansion (PCE) or any other reduced order model (ROM) technique (case dependent).

D) Data mining. Create scatter plots and box plots that show trends in the inputs/outputs and statistical relevance. In this way, the QoI may be used to answer a specific question of interest.

The computational approach used will now be described in more detail.

Numerical code verification is executed following Section 2 of [12], for a 2D Poiseuille and a 3D Womersley flow problem in a cylindrical tube. These problems have non-trivial analytical solutions that are used as true value. For both cases the discretisation error is monitored as the grid is systematically refined by halving as in [13]. If the ratio between mesh subdivisions is defined as $r_{i,j} = r_i/r_j$, then $r_{1,2} = r_{2,3} = r = 2.0$, a figure considerably larger than 1.3, the minimum value recommended [12]. The velocity field is the quantity of interest (QoI) to be verified as it is also the raw variable used obtained from the numerical model.

It needs to be established that the computational mesh will converge properly. For instance, the root mean square error (RMSE) between the solution i and j is defined through the L2 norm $\|.\|_2$ as:

$$\epsilon_{i,j} = \left\| v_i - v_j \right\| = \sqrt{(\tilde{v}_i - \tilde{v}_j)^2} \qquad (1)$$

Once all the cases are computed and the errors $\varepsilon_{1,2}$ and $\varepsilon_{2,3}$ computed the observed order of convergence is calculated as [14]:

$$s_{i,k} = 1 \cdot sign\left(\epsilon_{i,j}/\epsilon_{i,j}\right) \qquad (2)$$

$$q_{i,k}(p_{i,k}) = \ln\left(\frac{r_{j,k}^{p_{i,k}} - s}{r_{i,j}^{p_{i,k}} - s}\right) \tag{3}$$

$$p_{i,k} = [1/\ln(r_{j,k})]\left[\ln|\epsilon_{i,j}/\epsilon_{j,k}| + q_{i,k}(p_{i,k})\right] \tag{4}$$

Note that in the present case $r_{i,j} = r_{j,k} = r = 2.0$, so $q_{i,k}(p_{i,k}) = 0:0$ and therefore the previous system of equations is reduced to:

$$p_{i,k} = \frac{\ln|\epsilon_{i,j}/\epsilon_{j,k}|}{\ln(r)} \tag{5}$$

For three velocity fields computed $u_1$, $u_2$, $u_3$; $u_1$ being the coarsest, for three subdivision levels, the order of the convergence of the numerical scheme is given by:

$$p = \frac{\ln(\|u_1 - u_2\|_2/\|u_2 - u_3\|_2)}{\ln(2.0)} \tag{6}$$

With the observed p value, the grid convergence index (GCI) can be computed as [14]:

$$GCI_{i,j}^{95\%} = 1.25\frac{\epsilon_{i,j}}{r_{i,j}^{p} - 1} \tag{7}$$

This uncertainty estimate provides an interval $f \pm U^{95\%}$ within the true mathematical value fr falls with a probability of 95%.

In implementation, the VVUQ plan can be used to describe the application of a particular model (as well as providing a basis for determining whether the model can be used effectively). An example for LVAD may be as follows:

Question of Interest: For an apically implanted LVAD, does the selected pump speed produce: (a) complete aortic valve opening (QAO > 5[cm$^3$/s]); and (b) a Cardiac output compatible with life $(Q_{Ao}^{avg} + Q_{LVAD}^{avg} > 100[\text{cm}^3/\text{s}]$ for a range of HR and EF covering a HF patient population.

Context of Use (CoU): As previously indicated, the heart-LVAD computational model may be used to assist in the preclinical design and development of LVAD, by characterising aortic root, LVAD and intra-LV flows for a given pump speed.

Quantities of Interest (QoI): The QoIs used during the validation are the maximum and average flows through the outlet boundaries (LVAD flow $Q_{VAD}$ and aortic root flow $Q_{Ao}$).

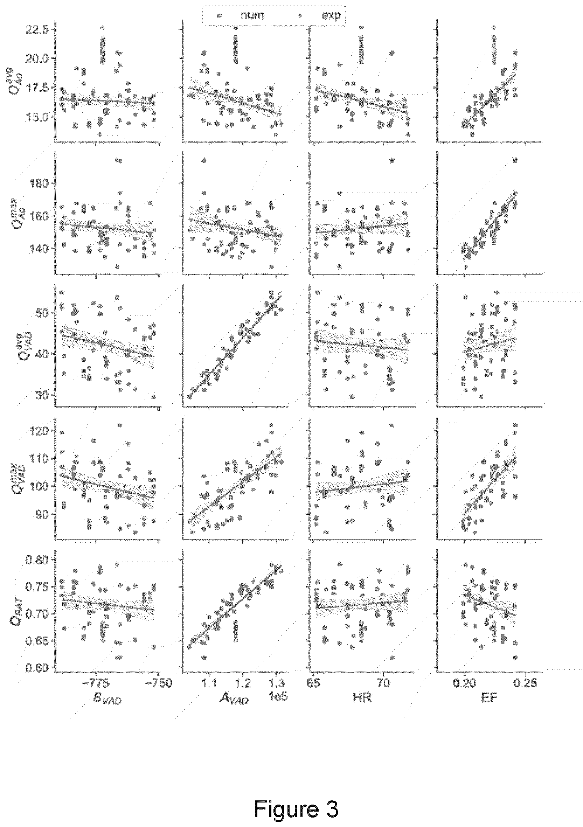

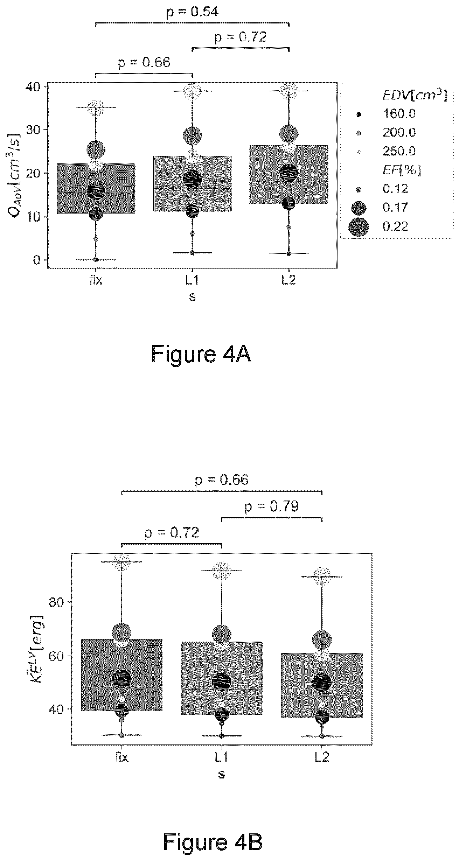

The model provides information about stagnation regions in the domain. Here, a population of 9 subjects with different EDVs and EF was treated with 3 speed modulation protocols (fix, L1, L2). The model shows no statistical difference of aortic flow and kinetic energy while there is a clear increase of the pulsatility index (PI). This shows that L1 and L2 might be less prone to thromboembolism than the fix treatment. This is a single example of the outputs of the tool. This is shown in Figures 3 and 4A to 4C.

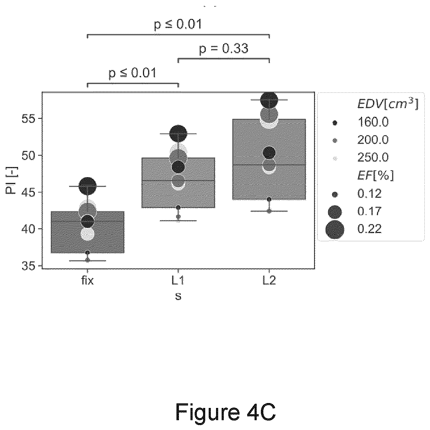

Figure 3 is a scatter plot showing a comparison between the numerical (blue) and experimental (orange) results. This shows a good correlation for most QoIs. Figures 4A to 4C show box and scatter plots for a population of 9 patients separated in three categories (fix, L1 and L2). While there is no statistical difference between them for aortic flow (Figure 4A) or for kinetic energy (Figure 4B) between the three categories there is a statistically significant increase of the pulsatility index (Figure 4C).

EXEMPLARY APPLICATIONS OF EMBODIMENTS OF THE DISCLOSURE

The disclosure describes, in its embodiments, a tool which from a given set of inputs that describe either a single patient or a patient population, and a VAD therapy (an LVAD therapy in the model specifically shown) can predict key operational indicators (such as indices for performance, safety and effectiveness). An exemplary and non- limiting set of applications are described as follows.

### 1. Optimisation of LVAD therapy for an uncertain population

A parameterised uncertain population may be described through probability distributions. Modelling may take place across a space defined by this parameterised population and a set of therapy parameters. This enables therapy parameters - for example, indices for safety, effectiveness and performance - to be optimised across the population. This can be used, for example, to create a preferred baseline for application of the therapy.

### 2. Surgical guidance of LVAD implantation position and configuration

For pre-defined patient parameters, it is possible to model optimised parameters for the LVAD implantation process. These may include implantation position and operation configuration. This process could be carried out in the planning stage for an operation to help establish an operation plan. In embodiments, such guidance could even be carried out during surgery - this may require a different computation strategy (for example, use of a reduced order model to accelerate calculation time).

### 3. Virtual ramp study (as in-the-box information)

The tool can be used to create tabulated data to be provided "in the box" with a VAD device - this can be used to determine the configuration of the device in the light of the patient's condition. For example, one critical parameter after LVAD implantation is the maximum pump speed that can be set (thus maximising cardiac output) while still allowing aortic valve opening (which is required to avoid long-term regurgitation). Such tabulated information could provide an initial value for the pump speed depending on the patient condition - this could reduce the amount of testing and optimisation time subsequently required.

### 4. Discovery and development of computational biomarkers

The tool can be used to discover, and subsequently develop, computational biomarkers and indicators to evolve parameterisation of VADs and LVADs. The goal of this development would typically be to develop and optimise device operation, with a view to maximising the likelihood of positive outcomes. Derivative markers may include statistical and risk indicators based on any combination of the quantities, function of quantities, and results (both directly and indirectly computed) from the tool - the practical utility provided by such biomarkers (in providing the most practically effective description of a system) will be a particular consideration in their development. Time-based and transient factors may prove significant here.

### 5. Precision predictive patient specific LVAD performance evaluation for clinician support

This may be either punctual or lifetime. Different approaches may be used in each case.

a. Punctual. Sensitivity analysis may be carried out (using a number of cases) for patient specific physiological information inputs. VAD parameters may then be optimised to deliver the best scenario for the given patient at a precise moment in time - this may be done, for example, to minimise the risk of thrombus formation (residence time, etc.) and to minimise any structural impact of the VAD on the patient (for example, avoiding suction of the heart wall or forced valve opening, and limiting stresses and strains imposed on the ventricle or elsewhere). For a therapy to be implemented, this may involve scenario analysis considering plausible placement, appropriate cannula shapes, implant depth, pump velocity and pressure-related profiles. Physiological sensitivity may also be considered here.
b. Longitudinal (lifetime) prediction. The "punctual" approach above can be followed while considering heart failure deterioration paths. At present, this is an empirical process - it is known that heart failure becomes more likely with time, and that VAD operating conditions need to be updated, but there is no systematic process to achieve this. Using the tool, deterioration paths based on observed medical events can be used to establish a patient warning table linking potential future patient conditions to associated optimised operating parameters. Relevant biomedical markers can be used to create effective patient/device management guidelines adjusted for the patient and adapted to support clinical decision making.

REFERENCES

[1] American Society of Mechanical Engineers. Assessing Credibility of Computational Modeling through Verification and Validation: Application to Medical Devices - V V 40 - 2018. Asme V&V 40-2018, page 60, 2018.

[2] Carlo R Bartoli, David Zhang, Jooeun Kang, Samson Hennessy-Strahs, David Restle, Jessica Howard, Gretchen Redline, Christian Bermudez, Pavan Atluri, and Michael A Acker. Clinical and in vitro evidence that subclinical hemolysis contributes to lvad thrombosis. The Annals of thoracic surgery, 105(3):807{814, 2018.

[3] Gordon DO Lowe. Virchow's triad revisited: abnormal flow. Pathophysiology of haemostasis and thrombosis, 33(5-6):455{457, 2003.

[4] Joseph G Rogers, Francis D Pagani, Antone J Tatooles, Geetha Bhat, Mark S Slaughter, Emma J Birks, Steven W Boyce, Samer S Najjar, Valluvan Jeevanandam, Allen S Anderson, et al. Intrapericardial left ventricular assist device for advanced heart failure. New England Journal of Medicine, 376(5):451{460, 2017.

[5] Lorenzo Rossini, Oscar O Braun, Michela Brambatti, Yolanda Benito, Adam Mizeracki, Marissa Miramontes, Cathleen Nguyen, Pablo Martinez-Legazpi, Shone Almeida, Megan Kraushaar, et al. Intraventricular flow patterns in patients treated with left ventricular assist devices. ASAIO Journal, 2020.

[6] MS Slaughter. Heartmate ii investigators: Advanced heart failure treated with continuous-ow left ventricular assist device. N Engl J Med, 361:2241{2251, 2009.

[7] Kiat T Tan and Gregory YH Lip. Red vs white thrombi: treating the right clot is crucial. Archives of internal medicine, 163(20):2534{2535, 2003.

[8] David Varga-Szabo, Irina Pleines, and Bernhard Nieswandt. Cell adhesion mechanisms in platelets. Arteriosclerosis, thrombosis, and vascular biology, 28(3):403{412, 2008.

[9] Rui Zhao, Joie N Marhefka, Fangjun Shu, Samuel J Hund, Marina V Kameneva, and James F Antaki. Micro-flow visualization of red blood cell-enhanced platelet concentration at sudden expansion. Annals of biomedical engineering, 36(7):1130, 2008.

[10] Neidlin et al. Understanding the influence of left ventricular assist device inflow cannula alignment and the risk of intraventricular thrombosis" BioMed Eng OnLine (2021) 20:47 https://doi.org/10.1186/s12938-021-00884-6

[11] Ong et al. Numerical investigation of the effect of cannula placement on thrombosis, Theoretical Biology and Medical Modelling 2013, 10:35

[12] American Society of Mechanical Engineers, 2009. "Standard for Verification and Validation in Computational Fluid Dynamics and Heat Transfer: ASME V&V 20". The American Society of Mechanical Engineers (ASME).

[13] Houzeaux, G., de la Cruz, R., Owen, H., and Vazquez, M., 2013. "Parallel uniform mesh multiplication applied to a Navier-Stokes solver". Computers and Fluids, 80(1), pp. 142-151.

[14] Roache, P. J., 1998. Verification and validation in computational science and engineering, Vol. 895. Hermosa Albuquerque, NM.

[15] Roache, P. J., 2002. "Code verification by the method of manufactured solutions". J. Fluids Eng., 124(1), pp. 4-10.

[16] Pedley, T. J., and Luo, X., 1995. Fluid mechanics of large blood vessels. Shaanxi People's Press.

Effect of Timings of the Lavare Cycle on the Ventricular Washout in an In Vitro Flow Visualization Setup. THANANYA KHIENWAD. ASAIO 2021

Impact of LVAD Implantation Site on Ventricular Blood Stagnation, Prisco. ASAIO 2017

Left Ventricular Assist Device Inflow Cannula Angle and Thrombosis Risk. Chivukula. Circulations Heart failure.

A clinical method for mapping and quantifying blood stasis in the left ventricle. Rossini. Journal of Biomechanics. 2015.

**Claims**

1. A method of configuring a ventricular assist device, comprising:

   determining one or more parameters of a patient heart;
   determining one or more parameters of the ventricular assist device;
   using a mechanical and fluid dynamic model of at least a ventricle where the ventricular assist device is or is to be used, computing predicted quantities of interest for the patient heart from operating the ventricular assist device using the determined parameters; and
   varying the one or more parameters of the patient heart and/or the one or more parameters of the ventricular assist device and again computing the predicted quantities of interest, repeating this step until a set of determined parameters and associated predicted quantities of interest are developed to meet a predetermined configuration objective; and
   configuring the ventricular assist device according to the predetermined configuration objective.

2. The method of claim 1, wherein the configuration objective comprises one or more of the predicted quantities of interest meeting predetermined criteria.

3. The method of claim 2, wherein the predetermined criteria are indices for one or more of performance, safety and effectiveness.

4. The method of any preceding claim, wherein the parameters of a patient heart relate to an arbitrary patient heart in an uncertain population of patients.

5. The method of any preceding claim, wherein the parameters of a patient heart comprise one or more anatomical parameters relating to a patient's anatomical geometry or cardiac activity.

6. The method of claim 5, wherein said one or more anatomical parameters comprise one or more of heart morphology, a ventricular volume, an ejection fraction and a heart rate.

7. The method of any preceding claim, wherein the parameters of a patient heart comprise one or more systemic parameters relating to a patient's system condition.

8. The method of claim 7, wherein the one or more systemic parameters comprise one or more of an arterial main pressure, an arterial resistance, and an arterial capacitance.

9. The method of any preceding claim, wherein the one or more parameters of the ventricular assist device comprises pump operation condition parameters.

10. The method of claim 9, wherein the one or more pump operation condition parameters comprise one or more of pump speed waveform, pump synchronisation with native heart rate, and a pump performance function.

11. The method of any preceding claim, wherein the quantities of interest comprise one or more quantities derived from the computation.

12. The method of claim 11, wherein the quantities of interest comprise one or more of residence time, velocity magnitude, kinetic energy, distortion time and pulsatility index.

13. The method of claim 12, wherein the predetermined condition relates to the likelihood of thrombus formation.

14. The method of any preceding claim, wherein computing the predicted quantities of interest comprises modelling over a first solid mechanics mesh determined from an original heart geometry and a second fluid dynamics mesh created by extruding inlets and outlets for flow development.

15. The method of claim 15, wherein computing the predicted quantities involves solving the Navier-Stokes equations.

Figure 1

Figure 2

Figure 3

Figure 4A

Figure 4B

Figure 4C

Europäisches Patentamt

European Patent Office

Office européen des brevets

# EUROPEAN SEARCH REPORT

**Application Number**

EP 21 38 3010

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | EP 3 848 938 A1 (KONINKLIJKE PHILIPS NV [NL]) 14 July 2021 (2021-07-14)<br>* paragraphs [0060] – [0068] *<br>* paragraph [0077]; figures 2-4 *<br>* paragraphs [0088] – [0089] *<br>* paragraphs [0106] – [0115] *<br>----- | 1-13,15<br><br>14 | INV.<br>A61M60/178<br>A61M60/50<br>G16H20/40<br>G16H40/60<br>G16H50/50 |
| X | US 2021/145361 A1 (DEL ALAMO DE PEDRO JUAN CARLOS [US] ET AL)<br>20 May 2021 (2021-05-20)<br>* paragraph [0080] *<br>* paragraphs [0185] – [0192] *<br>----- | 1-3,5-9,<br>11-13 | |
| X | WO 2018/108276 A1 (SINTEF TTO AS [NO])<br>21 June 2018 (2018-06-21)<br>* claims 1,6-9 *<br>* page 6, line 33 – page 7, line 5 *<br>----- | 1,15 | |
| X | US 2003/032853 A1 (KORAKIANITIS THEODOSIOS [US]) 13 February 2003 (2003-02-13)<br>* figure 28 *<br>* claims 1,2 *<br>* paragraph [0178] *<br>----- | 1 | TECHNICAL FIELDS SEARCHED (IPC)<br><br>A61M<br>G16H |

The present search report has been drawn up for all claims

| Place of search | Date of completion of the search | Examiner |
|---|---|---|
| The Hague | 20 April 2022 | Ingelbrecht, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 38 3010

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2022

| Patent document cited in search report | | Publication date | Patent family member(s) | | Publication date |
|---|---|---|---|---|---|
| EP 3848938 | A1 | 14-07-2021 | NONE | | |
| US 2021145361 | A1 | 20-05-2021 | US 2021145361 | A1 | 20-05-2021 |
| | | | WO 2019195783 | A1 | 10-10-2019 |
| WO 2018108276 | A1 | 21-06-2018 | CN 110268478 | A | 20-09-2019 |
| | | | EP 3555778 | A1 | 23-10-2019 |
| | | | ES 2836104 | T3 | 24-06-2021 |
| | | | JP 7021224 | B2 | 16-02-2022 |
| | | | JP 2020502681 | A | 23-01-2020 |
| | | | WO 2018108276 | A1 | 21-06-2018 |
| US 2003032853 | A1 | 13-02-2003 | AU 2001298042 | A1 | 06-05-2003 |
| | | | CA 2439390 | A1 | 01-05-2003 |
| | | | US 2003032853 | A1 | 13-02-2003 |
| | | | US 2003092961 | A1 | 15-05-2003 |
| | | | US 2004097782 | A1 | 20-05-2004 |
| | | | WO 03034893 | A2 | 01-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Assessing Credibility of Computational Modeling through Verification and Validation: Application to Medical Devices - V V 40 - 2018. Asme V&V, 2018, 60
- **CARLO R BARTOLI ; DAVID ZHANG ; JOOEUN KANG ; SAMSON HENNESSY-STRAHS ; DAVID RESTLE ; JESSICA HOWARD ; GRETCHEN REDLINE ; CHRISTIAN BERMUDEZ ; PAVAN ATLURI ; MICHAEL A ACKER.** Clinical and in vitro evidence that subclinical hemolysis contributes to lvad thrombosis. *The Annals of thoracic surgery,* 2018, vol. 105 (3), 807-814
- **GORDON DO LOWE.** *Virchow's triad revisited: abnormal flow. Pathophysiology of haemostasis and thrombosis,* 2003, vol. 33 (5-6), 455-457
- **JOSEPH G ROGERS ; FRANCIS D PAGANI ; ANTONE J TATOOLES ; GEETHA BHAT ; MARK S SLAUGHTER ; EMMA J BIRKS ; STEVEN W BOYCE ; SAMER S NAJJAR ; VALLUVAN JEEVANANDAM ; ALLEN S ANDERSON et al.** Intrapericardial left ventricular assist device for advanced heart failure. *New England Journal of Medicine,* 2017, vol. 376 (5), 455-457
- **LORENZO ROSSINI ; OSCAR O BRAUN ; MICHELA BRAMBATTI ; YOLANDA BENITO ; ADAM MIZERACKI ; MARISSA MIRAMONTES ; CATHLEEN NGUYEN ; PABLO MARTINEZ-LEGAZPI ; SHONE ALMEIDA ; MEGAN KRAUSHAAR et al.** Intraventricular flow patterns in patients treated with left ventricular assist devices. *ASAIO Journal,* 2020
- **MS SLAUGHTER.** Heartmate ii investigators: Advanced heart failure treated with continuous-ow left ventricular assist device. *N Engl J Med,* 2009, vol. 361, 2241-2251
- **KIAT T TAN ; GREGORY YH LIP.** Red vs white thrombi: treating the right clot is crucial. *Archives of internal medicine,* 2003, vol. 163 (20), 2534-2535
- **DAVID VARGA-SZABO ; IRINA PLEINES ; BERNHARD NIESWANDT.** Cell adhesion mechanisms in platelets. *Arteriosclerosis, thrombosis, and vascular biology,* 2008, vol. 28 (3), 403-412

- **RUI ZHAO ; JOIE N MARHEFKA ; FANGJUN SHU ; SAMUEL J HUND ; MARINA V KAMENEVA ; JAMES F ANTAKI.** Micro-flow visualization of red blood cell-enhanced platelet concentration at sudden expansion. *Annals of biomedical engineering,* 2008, vol. 36 (7), 1130
- **NEIDLIN et al.** Understanding the influence of left ventricular assist device inflow cannula alignment and the risk of intraventricular thrombosis. *BioMed Eng OnLine,* 2021, vol. 20, 47
- **ONG et al.** Numerical investigation of the effect of cannula placement on thrombosis. *Theoretical Biology and Medical Modelling,* 2013, vol. 10, 35
- Standard for Verification and Validation in Computational Fluid Dynamics and Heat Transfer: ASME V&V 20. American Society of Mechanical Engineers. The American Society of Mechanical Engineers (ASME), 2009
- **HOUZEAUX, G. ; DE LA CRUZ, R. ; OWEN, H. ; VAZQUEZ, M.** Parallel uniform mesh multiplication applied to a Navier-Stokes solver. *Computers and Fluids,* 2013, vol. 80 (1), 142-151
- **ROACHE, P. J.** Verification and validation in computational science and engineering. Hermosa Albuquerque, 1998, vol. 895
- **ROACHE, P. J.** Code verification by the method of manufactured solutions. *J. Fluids Eng.,* 2002, vol. 124 (1), 4-10
- **PEDLEY, T. J. ; LUO, X.** Fluid mechanics of large blood vessels. Shaanxi People's Press, 1995
- **THANANYA KHIENWAD.** Effect of Timings of the Lavare Cycle on the Ventricular Washout in an In Vitro Flow Visualization Setup. *ASAIO,* 2021
- **PRISCO.** Impact of LVAD Implantation Site on Ventricular Blood Stagnation. *ASAIO,* 2017
- **CHIVUKULA.** Left Ventricular Assist Device Inflow Cannula Angle and Thrombosis Risk. *Circulations Heart failure*
- **ROSSINI.** A clinical method for mapping and quantifying blood stasis in the left ventricle. *Journal of Biomechanics,* 2015